Europäisches Patentamt

European Patent Office

Office européen des brevets

(11) Veröffentlichungsnummer: **0 499 243 A1**

## EUROPÄISCHE PATENTANMELDUNG

(21) Anmeldenummer: **92102395.8**

(51) Int. Cl.5: **A61B 17/00**, A61B 19/00

(22) Anmeldetag: **13.02.92**

(30) Priorität: **14.02.91 DE 9101684 U**

(43) Veröffentlichungstag der Anmeldung:
**19.08.92 Patentblatt 92/34**

(84) Benannte Vertragsstaaten:
**PT**

(71) Anmelder: **Sterimed Gesellschaft für medizinischen Bedarf mbH
Fasanerieweg 13-17 Postfach 215
W-6600 Saarbrücken 3(DE)**

(72) Erfinder: **Scarfi, Andrea
Lindauer Strasse 46
W-7750 Konstanz(DE)**

(74) Vertreter: **Rupp, Herbert, Dr. et al
Byk Gulden Lomberg Chemische Fabrik
GmbH, Byk-Gulden-Strasse 2, Postfach 10 03
10
W-7750 Konstanz(DE)**

(54) **Extraktionsbeutel für die endoskopische Chirurgie.**

(57) Vorrichtung zum Entfernen von Material aus dem Bauchraum bei der endoskopischen Chirurgie, dadurch gekennzeichnet, daß sie einen Beutel aus reißfestem elastischen Material mit einem länglichen Ansatzstück zum Herausziehen des Beutels aus dem Bauchraum umfaßt.

Fig. 6

EP 0 499 243 A1

## Technisches Gebiet

Die Erfindung betrifft eine Vorrichtung zum Entfernen von Material aus dem Bauchraum bei der endoskopischen Chirurgie.

## Stand der Technik

Bei der endoskopischen Chirurgie müssen sehr oft Gewebeproben oder krankhaftes Gewebe aus dem Bauchraum entfernt werden. Das übliche Verfahren ist die Absaugung, gegebenenfalls unter vorheriger Zerkleinerung des Gewebes, oder die Entfernung als Ganzes mit einer Faßzange durch eine Trokarhülse. Bei Tumoren besteht dabei die Gefahr, daß Reste zurückbleiben und sich weiter verbreiten können. Bei der Entnahme von Proben ist vor allem bei der Zerkleinerung die Gefahr der Verunreinigung und Verstreuung von Gewebeteilen im Bauchraum gegeben. Hinzu kommt, daß das Zerkleinern sehr mühsam und zeitaufwendig ist.

## Beschreibung der Erfindung

Der vorliegenden Erfindung liegt nun die Aufgabe zugrunde, eine Vorrichtung zu schaffen, mit der gefahrlos im Bauchraum zu entfernendes Gewebe eingesammelt und aus dem Bauchraum entfernt werden kann.

Erfindungsgemäß gelöst wird diese Aufgabe durch eine Vorrichtung, die dadurch gekennzeichnet ist, daß sie einen Beutel aus reißfestem elastischen Material mit einem länglichen Ansatzstück zum Herausziehen des Beutels aus dem Bauchraum umfaßt.

Gegenstand der Erfindung ist daher eine Vorrichtung zum Entfernen von Material aus dem Bauchraum bei der endoskopischen Chirurgie, dadurch gekennzeichnet, daß sie einen Beutel aus reißfestem elastischen Material mit einem länglichen Ansatzstück zum Herausziehen des Beutels aus dem Bauchraum umfaßt.

Weitere Gegenstände der Erfindung ergeben sich aus den Patentansprüchen.

Das mit dem Beutel fest verbundene Ansatzstück ist als Lasche, vorzugsweise jedoch als Stab oder Rohr ausgebildet. Die Länge des Ansatzstückes ist so bemessen, daß alle Teile der Bauchhöhle erreicht werden können und dabei das Ansatzstück proximal in einer genügenden Länge aus der Bauchhöhle ragt, um dem Operateur Manipulationen zu erlauben. Das Ansatzstück weist demnach eine Länge von 30 bis 60 cm, vorzugsweise etwa 40 bis 50 cm auf. Wenn das Ansatzstück als Stab oder Rohr ausgebildet ist, reicht das Ansatzstück vorzugsweise in den Beutel hinein, wobei auch der in den Beutel hineinragende Teil des Ansatzstücks mit diesem zumindest teilweise unlösbar verbunden ist. In einer besonderen Ausführungsform ragt das rohr- oder stabförmige Ansatzstück bis auf den Boden des Beutels.

Der Durchmesser des rohr- bzw. stabförmigen Ansatzstückes entspricht dem Durchmesser wie er bei Geräten für die endoskopische Chirurgie üblich ist und beträgt in der Regel 2 bis 5, vorzugsweise 2,5 bis 3,5 mm.

Der Beutel ist beispielsweise sackartig gestaltet, d.h. er weist einen im wesentlichen rechteckigen Querschnitt auf. Der Boden des Beutels kann auch halbkugelförmig gestaltet sein. In einer bevorzugten Ausführungsform ist der Beutel im wesentlichen wie eine Spitztüte, d.h. in Richtung Boden im wesentlichen konisch zulaufend gestaltet. Besonders bevorzugt sind Beutel, deren oberer Rand vom Ansatzstück schräg nach unten verläuft. Das Fassungsvermögen des Beutels ist an den beabsichtigten Zweck angepaßt und beträgt beispielsweise zwischen 20 und 300 ml, vorzugsweise 40 bis 150 ml.

Der Beutel ist vorzugsweise verschließbar ausgeführt. Zweckmäßigerweise erfolgt das Verschließen des Beutels über einen Zugfaden, der in einem Tunneldurchzug am oberen Rand des Beutels verläuft. Durch Ziehen an den beiden freien Enden des Zugfadens kann der Beutel verschlossen werden. Der Zugfaden ist vorzugsweise so lang, daß seine freien Enden mindestens bis an das proximale Ende des Ansatzstückes reichen, so daß der Operateur den Beutel durch Ziehen an den beiden freien Enden des Zugfadens schließen kann. Es ist auch möglich, ein Ende des Zugfadens in Höhe des Beutelrandes am Ansatzstück zu fixieren. In diesem Fall läßt sich der Beutel durch Ziehen am anderen freien Ende zuziehen. Der Zugfaden weist zumindest in dem Bereich, der sich im Tunneldurchzug des oberen Beutelrandes befindet, eine Steifigkeit auf, die den oberen Beutelrand offenhält, solange nicht am freien bzw. den freien Enden des Zugfadens Zug ausgeübt wird. Alternativ kann der obere Rand des Beutels so verstärkt sein, daß die Beutelöffnung in Ruhestellung offensteht. Im Falle eines stab- oder rohrförmigen Ansatzstückes kann dieses ein oder zwei Längsrillen aufweisen, in denen das bzw. die freien Enden des Zugfadens zum proximalen Ende des Ansatzstückes geführt werden. Im Falle eines rohrförmigen Ansatzstückes kann das bzw. können die freien Enden des Zugfadens im Innenlumen des Ansatzstückes geführt werden. Zur besseren Führung des bzw. der freien Enden des Zugfadens entlang dem Ansatzstück kann eine über das Ansatzstück geschobene schlauchartige oder rohrförmige Hülse gezogen sein, die bis kurz vor den oberen Rand des Beutels reicht. Die erfindungsgemäße Vorrichtung wird aus den in der medizinischen Technik üblichen Materialien hergestellt, wobei für den Beutel und den Zugfaden möglichst reißfestes Material verwendet werden sollte. Das rohr- oder stabförmige Ansatzstück wird aus Kunststoff oder Metall gefertigt, wobei Kunststoff bevorzugt ist. Der Zugfaden kann auch als kunststoffummantelte Drahtlitze gestaltet sein.

Bei endoskopischen Operationen wird meist zu Beginn eine relativ großlumige Trokarhülse in die Bauchdecke eingeführt. Diese erste Trokarhülse weist im Bereich des proximalen Endes einen Ventilschieber auf, der beispielsweise durch Betätigen einer seitlichen Druckplatte gegen eine Rückstellfeder geöffnet werden kann. Durch diese verschließbare erste Trokarhülse werden die im Verlauf der Operation benötigten Geräte eingeführt. Hierzu wird das benötigte Gerät, z.B. eine Faßzange, in eine zweite Trokarhülse eingeführt, deren Außendurchmesser so an den Innendurchmesser der ersten Trokarhülse angepaßt ist, daß Dichtwirkung erzielt wird. Die zweite Trokarhülse weist am proximalen Ende einen Anschlußstutzen auf, auf den eine Dichtkappe mit zentralem Durchlaß gesteckt werden kann. Aus einem Standardsatz von Dichtkappen wird diejenige ausgewählt, deren zentraler Durchlaß den Stamm des einzuführenden Geräts dichtend umschließt, ohne dessen Verschiebbarkeit allzusehr zu behindern.

Das Einbringen der erfindungsgemäßen Vorrichtung, bei der das Ansatzstück des Beutels als Lasche ausgebildet ist, in die Bauchhöhle kann erfolgen, indem der Beutel über ein Ende eines Stabes oder Rohres gezogen wird. Das proximale Ende des Stabes oder Rohres wird zusammen mit dem proximalen Ende der Lasche und gegebenenfalls den freien Enden des Zugfadens durch das distale Ende einer oben erwähnten zweiten Trokarhülse so weit eingeführt, bis der Beutel vollständig innerhalb der zweiten Trokarhülse liegt. Über den Stab bzw. das Rohr und die Lasche wird sodann von proximal her eine passende Dichtungskappe gezogen und auf den proximalen Anschlußstutzen gesteckt. Die zweite Trokarhülse wird jetzt in die bereits durch die Bauchdecke laufende erste Trokarhülse eingeführt. Das Rohr bzw. der Stab kann nun nach distal verschoben werden, um den Beutel in die gewünschte Position zu bringen.

Der Beutel kann jetzt, soweit sich der Beutel durch materialbedingte Rückstellkräfte nicht von selbst entfaltet, beispielsweise mit Hilfe einer durch einen zweiten Zugang in die Bauchhöhle eingeführten Faßzange entfaltet bzw. vom Ende des Einführungsstabes abgewickelt werden. Nach dem Befüllen mit Gewebeteilen wird der Beutel gegebenenfalls über den Zugfaden verschlossen und am laschenförmigen Ansatzstück gegen das distale Ende der zweiten Trokarhülse gezogen. Wenn der Befüllungsgrad des Beutels es zuläßt, wird er in die zweite Trokarhülse gezogen und zusammen mit dieser aus der Bauchhöhle entfernt. Falls er in der zweiten Trokarhülse nicht Platz hat, werden die beiden Trokarhülsen zusammen mit dem Beutel aus dem Operationseinschnitt gezogen. Falls der gefüllte Beutel wegen seines zu großen Durchmessers nicht durch den Operationseinschnitt herausgezogen werden kann, kann der Einschnitt entsprechend erweitert werden. Alternativ wird nur der obere Rand des Beutels durch den Operationseinschnitt gezogen, so daß der sich noch in der Bauchhöhle befindliche Beutel von außen geleert werden kann, indem der Inhalt portionsweise, nötigenfalls nach vorheriger mechanischer Zerkleinerung, soweit entnommen wird, bis sich der Beutel durch den Operationseinschnitt entfernen läßt. Besteht der Inhalt des Beutels z.B. aus einer voluminösen, Flüssigkeit enthaltenden Cyste, so kann diese angestochen und die ausfließende Flüssigkeit abgesaugt werden und anschließend kann der nichtflüssige Teil des Beutelinhalts bei Bedarf noch zerkleinert werden.

Das Vorgehen mit einer erfindungsgemäßen Vorrichtung, bei der das Ansatzstück des Beutels stab- oder rohrförmig gestaltet ist, geschieht in entsprechender Weise. Bei einem rohrförmigen Ansatzstück kann im Bedarfsfall auf das proximale Ende ein Anschlußstutzen, beispielsweise ein Luer-Lock-Ansatz, gesteckt werden, an den z.B. eine Absaugeinrichtung angeschlossen werden kann. Gewünschtenfalls kann der Anschlußstutzen mit einer Verschlußkappe verschlossen werden.

In Zusammenhang mit der vorliegenden Erfindung ist unter distal untersucherfern und unter proximal untersuchernah zu verstehen.

Nachstehend soll die Erfindung anhand der Fig. 1 bis 6 näher erläutert werden.

Fig. 1 bis 4    zeigen schematische Ansichten verschiedener Ausführungsformen der erfindungsgemäßen Vorrichtung

Fig. 5    zeigt einen Querschnitt durch eine Ausführungsform

Fig. 6 und 7    illustrieren die Anwendung einer erfindungsgemäßen Vorrichtung

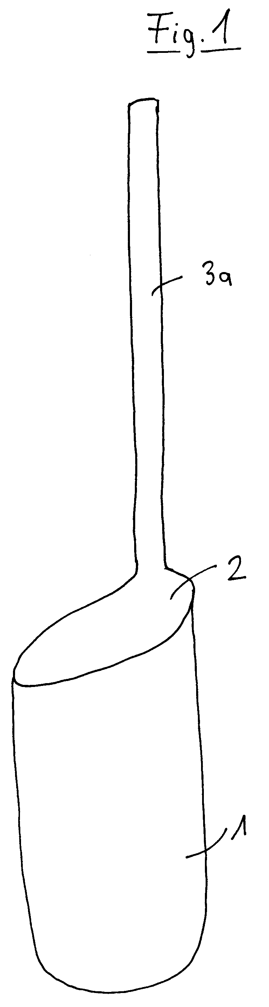

In der Fig. 1 ist die einfachste Ausführungsform einer erfindungsgemäßen Vorrichtung dargestellt. Sie besteht aus einem sackartigen Beutel 1 an dessen oberen Rand ein Ansatzstück 3 in Gestalt einer länglichen Lasche 3a angebracht ist.

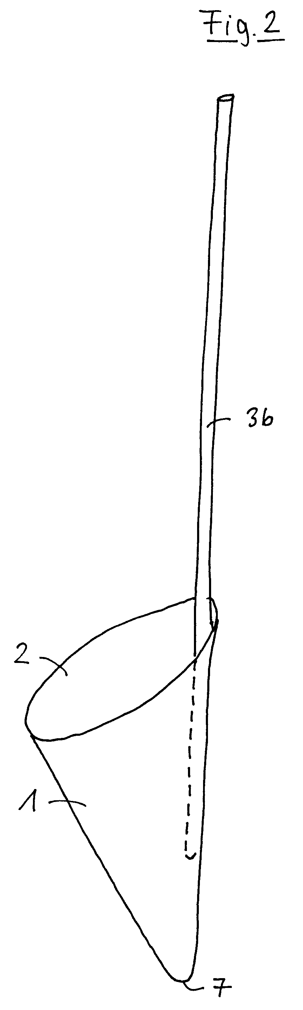

Die Fig. 2 zeigt eine Ausführungsform, bei der der Beutel 1 sich in Richtung auf seinen Boden 7 konisch verjüngt und damit eine köcherartige Form aufweist. In das Innere des Beutels 1 ragt das fest mit dem Beutel verbundene distale Ende eines als Stab 3b ausgebildeten Ansatzstückes 3.

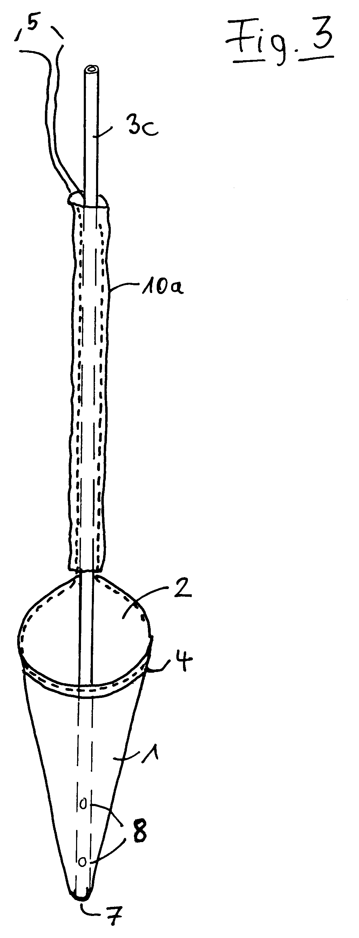

Bei der in Fig. 3 dargestellten Ausführungsform ist das Ansatzstück 3 als Rohr 3c ausgebildet. Das Rohr 3c ragt bis auf den Boden 7 des Beutels 1 und weist im Bereich seines in den Beutel 1 ragenden distalen Endes seitliche Öffnungen 8 auf. Das obere Ende des Beutels 1 weist einen sogenannten Tunnelzug 4 auf, d.h. der obere Rand des Beutels 1 ist als Hohlsaum ausgebildet, in dem ein Zugfaden 5 verläuft. Durch Ausüben von Zug an den freien Enden des Zugfadens kann die Öffnung des Beutels 1 verschlossen werden. Die beiden freien Enden des Zugfadens 5 sind entlang des Stabes 3c nach distal geführt, wobei Stab 3c und die beiden freien Enden des Zugfadens 5 teilweise von einer schlauchartigen Hülse 10a umgeben sind.

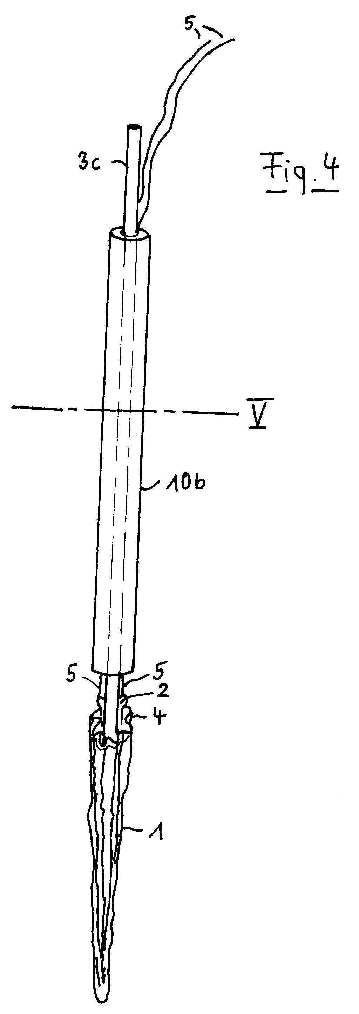

Bei der Ausführungsform nach Fig. 4 ist der Beutel 1 um das in den Beutel 1 hineinragende distale Ende des Rohrs 3c gefaltet. Seine Öffnung 2 ist durch Zug an den freien Enden des im Tunneldurchzug 4 verlaufenden Zugfadens 5 verschlossen. Die freien Enden des Zugfadens 5 verlaufen entlang des rohrförmigen Ansatzstücks 3c nach proximal. Eine rohrförmige Hülse 10b umschließt das rohrförmige Ansatzstück 3c und die freien Enden des Zugfadens 5 etwa ab oberhalb des oberen Rands des Beutels 1. Nach proximal ragen das rohrförmige Ansatzstück 3c und die freien Enden des Zugfadens 5 soweit aus der rohrförmigen Hülse 10b heraus, daß genügend Freiraum für eine Manipulation des rohrförmigen Ansatzstückes 3c und des Zugfadens 5 verbleibt. In dem in Fig. 4 dargestellten Zustand kann die erfindungsgemäße Vorrichtung durch eine übliche Trokarhülse (nicht dargestellt) in den Bauchraum des Patienten eingeführt werden. Auf das proximale Ende des Rohrs 3c kann bei Bedarf z.B. ein Luer-Lock Ansatz aufgesteckt werden, um damit Konnektierungsmöglichkeiten, z.B. mit einer Saugeinrichtung, zu schaffen. Bei Bedarf kann eine Verschlußkappe vorgesehen werden.

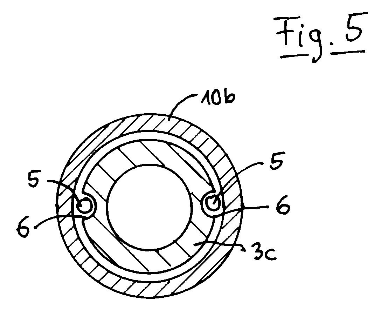

Fig. 5 zeigt einen schematischen Querschnitt durch eine erfindungsgemäße Vorrichtung entlang der Linie V in Fig. 4. Die freien Enden des Zugfadens 5 liegen hier in Längsrillen 6 des in der rohrförmigen Hülse 10b verlaufenden rohrförmigen Ansatzstückes 3c.

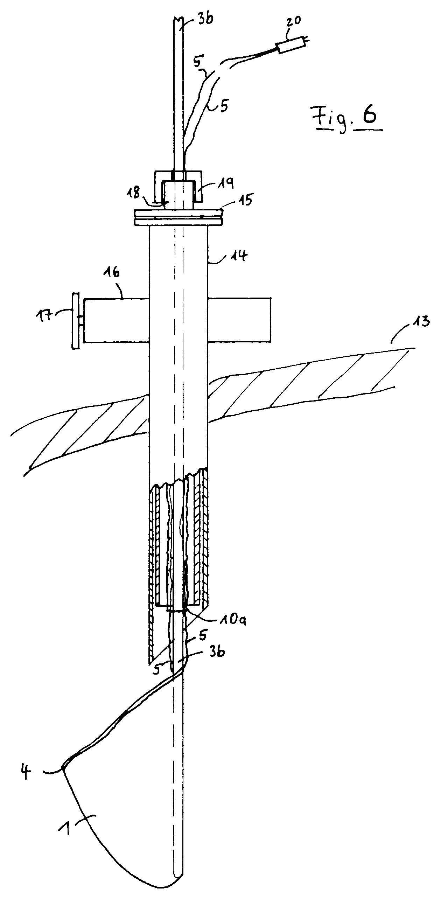

In Fig. 6 ist schematisch die Anwendung einer erfindungsgemäßen Vorrichtung bei einer endoskopischen Operation dargestellt. In eine erste durch die Bauchdecke 13 geführte Trokarhülse 14, die einen über eine Druckplatte 17 betätigbaren Ventilschieber 16 aufweist, ist eine zweite Trokarhülse 15 von proximal her eingeführt. Durch einen Anschlußstutzen 18 mit aufgesetzter Dichtkappe 19 am proximalen Ende der zweiten Trokarhülse 15 verlaufen das stabförmige Ansatzstück 3b und die freien Enden des Zugfadens 5 einer erfindungsgemäßen Vorrichtung. Das stabförmige Anschlußstück 3b ist oberhalb des Beutels 1 mit einer schlauchartigen Hülse 10a umgeben, in der auch die beiden Enden des durch den Tunnelzug 4 des Beutels 1 gezogenen Zugfadens 5 verlaufen. Das stabförmige Anschlußstück 3b ist bereits so weit nach distal in die zweite Trokarhülse 15 verschoben, daß sich der beim Einführen der zweiten Trokarhülse 15 in die erste Trokarhülse 14 innerhalb der zweiten Trokarhülse 15 befindliche Beutel 1 frei entfalten läßt und damit für die Aufnahme von zu entfernendem Material bereit ist. Nach dem Befüllen des Beutels 1 kann dieser durch Zug an den durch eine Klemmhülse 20 zusammengehaltenen Enden des Zugfadens 5 geschlossen werden.

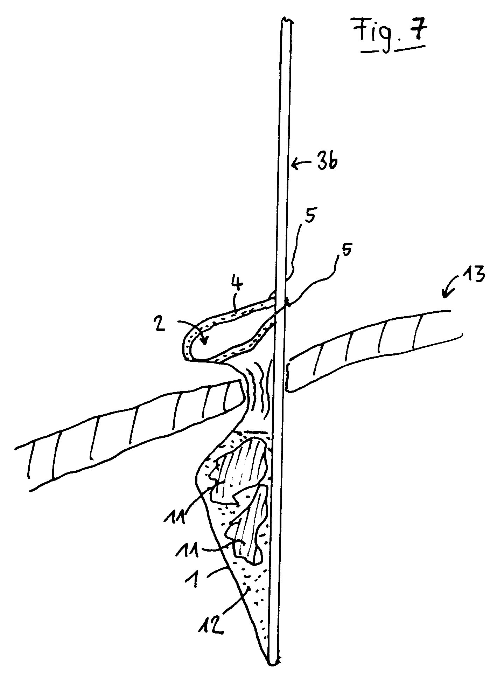

Fig. 7 zeigt schematisch den distalen Teil einer erfindungsgemäßen Vorrichtung, nachdem im Laufe eines laparoskopisch durchgeführten Eingriffs Beutel 1 mit Gewebeteilen 11 und flüssigem Material 12 gefüllt wurde und das stabförmige Ansatzstück 3b anschließend soweit nach proximal verbracht wurde, bis die Öffnung 2 des Beutels 1 außerhalb der Bauchdecke 13 zu liegen kommt. Nach dem Öffnen der Öffnung 2 des Beutels 1 durch Nachlassen des Zugfadens 5 kann nun der Inhalt des Beutels entnommen werden. Nicht absaugbare Teile werden z.B. mit Hilfe geeigneter Zangen, nötigenfalls nach vorheriger Zerkleinerung entnommen.

**Patentansprüche**

1. Vorrichtung zum Entfernen von Material aus dem Bauchraum bei der endoskopischen Chirurgie, dadurch gekennzeichnet, daß sie einen Beutel (1) aus reißfestem elastischen Material mit einem länglichen Ansatzstück (3) zum Herausziehen des Beutels (1) aus dem Bauchraum umfaßt.

2. Vorrichtung nach Patentanspruch 1, dadurch gekennzeichnet, daß das Ansatzstück (3) als Lasche (3a) ausgebildet ist.

3. Vorrichtung nach Patentanspruch 1, dadurch gekennzeichnet, daß das Ansatzstück (3) als Stab (3b) ausgebildet ist.

4. Vorrichtung nach Patentanspruch 1, dadurch gekennzeichnet, daß das stabförmige Ansatzstück (3) als Rohr (3c) ausgebildet ist.

5. Vorrichtung nach Patentanspruch 1, dadurch gekennzeichnet, daß die Öffnung (2) schräg zum länglichen Ansatzstück (3) geschnitten ist.

6. Vorrichtung nach Patentanspruch 1, dadurch gekennzeichnet, daß der Beutel (1) sackartig gestaltet ist.

7. Vorrichtung nach Patentanspruch 1, dadurch gekennzeichnet, daß sich der Beutel (1) in Richtung auf seinen Boden (7) konisch verjüngt.

8. Vorrichtung nach Patentanspruch 1, dadurch gekennzeichnet, daß der obere Rand des Beutels (1) als Tunneldurchzug (4) mit eingezogenem Zugfaden (5) ausgebildet ist.

9. Vorrichtung nach Patentanspruch 8, dadurch gekennzeichnet, daß der Zugfaden (5) aufgrund seiner Steifigkeit den Beutel (1) offenhält.

10. Vorrichtung nach Patentanspruch 8, dadurch gekennzeichnet, daß die freien Enden des Zugfadens (5) innerhalb einer schlauchartigen oder rohrförmigen Hülse (10a; 10b), die das längliche Ansatzstück (3) umgibt, verlaufen.

11. Vorrichtung nach Patentanspruch 3 oder Patentanspruch 4, dadurch gekennzeichnet, daß das als Stab (3b) oder Rohr (3c) ausgebildete Ansatzstück (3) in den Beutel (1) hineinreicht.

12. Vorrichtung nach Patentanspruch 11, dadurch gekennzeichnet, daß das als Stab (3b) oder Rohr (3c) ausgebildete Ansatzstück (3) bis auf den Boden (7) des Beutels (1) reicht.

13. Vorrichtung nach Patentanspruch 11, dadurch gekennzeichnet, daß das als Rohr (3c) ausgebildete Ansatzstück (3) mindestens eine seitliche Öffnung (8) im Bereich des Beutels (1) aufweist.

# Fig.1

Fig. 2

Fig. 3

Fig. 4

Fig. 5

Fig. 6

Fig. 7

Europäisches Patentamt

# EUROPÄISCHER RECHERCHENBERICHT

Nummer der Anmeldung

EP 92 10 2395

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-2 514 428 (PRÖPPER)<br><br>* Seite 1 - Seite 2; Abbildungen 1,2 *<br>--- | 1-4,6,<br>8-10 | A61B17/00<br>A61B19/00 |
| A | DE-C-25 796 (VERGUEIRO)<br><br>* Ansprüche 1,2; Abbildung 1 *<br>--- | 1,3,4,6,<br>8-10 | |
| A | DE-A-3 542 667 (WOLF)<br>* Anspruch 1; Abbildungen 1,2 *<br>--- | 1,6,8-10 | |
| A | EP-A-0 195 444 (KÖRBER)<br>* Anspruch 1; Abbildungen 3-6 *<br>--- | 1 | |
| X,P | EP-A-0 465 051 (VANCE)<br>* Zusammenfassung; Abbildungen 4-9 *<br>--- | 1 | |
| X,P | US-A-5 074 867 (WILK)<br>* Spalte 5, Zeile 5 - Spalte 6, Zeile 6; Abbildungen 2A-H *<br>----- | 1 | |

|  | RECHERCHIERTE SACHGEBIETE (Int. Cl.5) |
|---|---|
|  | A61B |

Der vorliegende Recherchenbericht wurde für alle Patentansprüche erstellt

| Recherchenort | Abschlußdatum der Recherche | Prüfer |
|---|---|---|
| DEN HAAG | 19 MAI 1992 | MOERS R. |

EPO FORM 1503 03.82 (P0403)